(19) Europäisches Patentamt
European Patent Office
Office européen des brevets

(11) Veröffentlichungsnummer: **0 002 755**
**B1**

(12) # EUROPÄISCHE PATENTSCHRIFT

(21) Anmeldenummer: **78101724.9**

(22) Anmeldetag: **16.12.78**

(51) Int. Cl.[3]: **C 07 C 148/00,**
C 07 C 149/28,
C 07 C 149/34,
C 07 C 149/415
//C07C149/40

(54) **Verfahren zur Herstellung von Arylthiolen und die so erhaltenen Verbindungen.**

(30) Priorität: **30.12.77 CH 16300/77**

(43) Veröffentlichungstag der Anmeldung:
**11.07.79 Patentblatt 79/14**

(45) Bekanntmachung des Hinweises auf die Patenterteilung:
**18.03.81 Patentblatt 81/11**

(84) Benannte Vertragsstaaten:
**CH DE FR GB IT**

(56) Entgegenhaltungen:
**DE - A - 1 543 946**
**US - A - 2 402 641**

**Chemical Abstracts, Band 88, Nr. 17, 24. April 1978 COLUMBUS, OHIO, USA Seite 517, Abstract Nr. 120 864y**

(73) Patentinhaber: **CIBA-GEIGY AG**
**Patentabteilung Postfach**
**CH-4002 Basel (CH)**

(72) Erfinder: **Thies, Hans, Dr.**
**Tulpenweg 9**
**D-7889 Rheinfelden/Degerfelden (DE)**

(72) Erfinder: **von Kaenel, Fred**
**Jurastrasse 19**
**CH-4411 Seltisberg (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 755 B1

# Verfahren zur Herstellung von Arylthiolen und die so erhaltenen Verbindungen

Die vorliegende Erfindung betrifft ein neues Verfahren zur Herstellung von Arylthiolen durch Hydrierung von Arylsulfochloriden in Gegenwart von Platin-Katalysatoren.

Es ist bekannt, dass Arylsulfinate, Arylthiosulfonate und Arylsulfochloride in Gegenwart von Sulfiden oder Polysulfiden des Co, Ni, Fe oder Mo bei Temperaturen von 150 bis 275°C und Drucken von 30 bis 170 bar zu Thiophenolen hydriert werden können (US--PS 2.402.641).

Im UdSSR—Patent 461.101 wird die Reduktion von 2-Naphthylsulfochlorid mit Wasserstoff in Gegenwart von Co-Polysulfid bei 130 bis 150°C und 50 bis 100 bar in Chlorobenzol beschrieben.

Weiterhin aus der DT—OS 19 03 968 bekannt, dass Diphenyldisulfid mit Wasserstoff bei 150 bar in Gegenwart von Ni- oder Co-Sulfid bzw. Polysulfid als Katalysator zum entsprechenden Thiophenol reduziert werden kann.

Gemäss niederländischer Patentanmeldung 66.11430 werden durch Hydrierung der Na-Salze der Benzolsulfinsäure und p-Toluolsulfinsäure in Form einer wässrigen Lösung bei Temperaturen von 125 bis 200°C und Drucken zwischen 30 und 105 bar mit Wasserstoff in Gegenwart von Platinsulfid auf Kohle als Katalysator die entsprechenden Thiophenole erhalten.

Ferner sei auch auf das selbst in der Literatur (Houben-Weyl, Meth. der Org.Chemie, 4.Aufl., Bd. IX, Seiten 29 ff) bezüglich des Erfindungsgegenstandes festgehaltene Vorurteil hingewiesen, dass bei der Reduktion von Sulfonsäurechloriden aus einer Suspension von Fe oder Zn, speziell bei höheren Temperaturen und in Gegenwart von Wasser (Reaktionswasser) nur mässige Ausbeuten an Thiophenolen erhalten werden, wobei für die Erniedrigung der Ausbeuten die Disproportionierung von Arylsulfinsäuren als Zwischenprodukt, die nach folgendem Schema verläuft, verantwortlich gemacht wird.

$$3\ ArSO_2H \rightarrow ArSO_2SAr + ArSO_3H + H_2O$$

$$5\ ArSO_2H \rightarrow Ar\text{-}S\text{-}S\text{-}Ar + 3ArSO_3H + H_2O$$

Die während der Disproportionierung entstehenden Sulfonsäuren können im Gegensatz zu Sulfonsäurechloriden nicht mehr zum Thiophenol reduziert werden.

Zusätzlich bilden sich dabei noch grosse Mengen an ökologisch bedenklichen, wieder aufzuarbeitenden Metallsalzen.

Alle genannten Verfahren weisen ausserdem eine Reihe von Nachteilen auf.

So verlaufen viele der genannten Umsetzungen über mehrere Stufen und liefern insbesondere bei den halogenierten Aromaten keine einheitlichen Produkte, was zu unbefriedigenden Ausbeuten führt und zusätzliche Reinigungsoperationen erforderlich macht. All diese Schwierigkeiten führten dazu, ein neues Verfahren zu suchen, das es gestattet, Arylthiole auf einfache Weise und in der geforderten Reinheit und Ausbeute herzustellen. Ueberraschenderweise wurde nun gefunden, dass bei einer Hydrierung von Arylsulfochloriden in organischen Lösungsmitteln in Gegenwart von Platin-Katalysatoren die genannten Nachteile entfallen und in einer einzigen Reaktionsstufe die entsprechenden Arylthiole mit einem hohen Reinheitsgrad und in hohen Reaktionsausbeuten erhalten werden. Insbesondere wird die Reduktionsreaktion durch Substituenten nicht negativ beeinflusst, z.B. wird an Aromaten gebundenes Chlor nicht ausgetauscht.

Das neue Verfahren zur Herstellung von Arylthiolen durch katalytische Hydrierung von Arylsulfochloriden in einem protonischen oder aprotonischen organischen Lösungsmittel bei erhöhtem Druck und unter erhöhten Temperaturen ist dadurch gekennzeichnet, dass man in Gegenwart von Platin als Katalysator bei Temperaturen zwischen 100 und 180°C, mit Wasserstoff unter einem Druck von 2 bis 140 bar, hydriert.

Bevorzugt verläuft das neue Verfahren in der Weiser, dass man zunächst mit 1 Mol Wasserstoff pro Mol Sulfochlorid bei Temperaturen von 20 bis 50°C vorhydriert und das Reaktionsgemisch erst anschliessend aufheizt. Die weitere Hydrierung wird zwischen 120 und 180°C, insbesondere 140 bis 150°C, und einem Wasserstoffdruckbereich von 20 bis 120 bar, insbesondere 30 bis 100 bar, durchgeführt.

Der Verlauf des erfindungsgemässen Verfahrens wird durch die folgende Gleichung wiedergegeben:

$$ArSO_2Cl + 3H_2 \rightarrow ArSH + 2H_2O + HCl.$$

Als Ausgangsverbindungen des erfindungsgemässen Verfahrens kommen einkernige und mehrkernige, unsubstituierte und substituierte Arylsulfochloride in Betracht. Als Beispiele von Substituenten seien die folgenden genannt: Halogen, insbesondere Chlor, Alkylgruppen, wie Aethyl und insbesondere Methyl, Alkoxygruppen, wie Methoxy, Alkylarylgruppen oder Aryloxygruppen. Namentlich erwähnt seien: Benzosulfochlorid, 4-Chlorbenzolsulfochlorid, 3-Methyl-4,6-dichlor-benzosulfochlorid, 3-Chlor-5-methyl-6-cyan-benzolsulfochlorid, 2-Naphthalinsulfochlorid, 1-Chlor-8-naphthalinsulfochlorid und 2,5-Dichlorbenzolsulfochlorid.

Als Katalysator wird zweckmässigerweise kommerziell zugängliches Platen auf einem Träger mit

hoher spezifischer Oberfläche verwendet. Solche Träger sind z.B. Aktivkohle, Kieselgel, Aluminiumgel oder Magnesiagel. Vorteilhaft werden 100 bis 2000 mg insbesondere 200 bis 400 mg, Katalysator pro Mol Arylsulfochlorid im Verfahren gemäss vorliegender Erfindung verwendet.

Als Lösungsmittel, die als Reaktionsmedium dienen, kommen vor allem solche in Betracht, in denen die Reaktionssubstrate und Produkte löslich und die reduktionsresistent sind. Statt einheitlicher Lösungsmittel können auch Mischungen verwendet werden.

Bevorzugte Lösungsmittel sind die niederen aliphatischen Alkohole, wie Isopropanol und Aethanol, aber auch z.B. Dimethylformamid, desweiteren aromatische Lösungsmittel, wie z.B. Toluol, Xylole und Chlorbenzole. In besonderen Fällen sind auch aliphatische Kohlenwasserstoffe bzw. deren Gemische, wie z.B. Leichtbenzin, geeignet.

Vorteilhaft verwendet man im erfindungsgemässen Verfahren ein Verhältnis von Arylsulfochlorid zu Lösungsmittel von 1:0,5 bis 1:20 und insbesondere von 1:2 bis 1:5.

Im einzelnen kann das Eintopfverfahren wie folgt durchgeführt werden:

Zu einer Lösung eines Arylsulfochlorids in einem polaren Lösungsmittel, wie z.B. Isopropanol, die sich in einem Doppelmantel-Druckkessel (max. Arbeitsdruck 150 bar) aus Hastelloy B® oder emailliertem Stahl befindet, und der mit einem Begasungsrührer (ca. 1500 UpM), Thermometer, Manometer, Ueberdruckventil mit Berstscheibe ausgerüstet ist, gibt man pro Mol Substrat ca. 4 bis 8 g Katalysator — 200 bis 400 mg Platin auf Kohle — hinzu. Danach wird der Innendruck mit Wasserstoff auf 5 bar gestellt und während 20 Minuten der Kessel und das Wasserstoff-Reservoir einer Dichtigkeitsprüfung unterzogen.

Die Vorhydrierung bei offenem Einlassventil (40 bar $H_2$) mit 1 Mol Wasserstoff/Mol Substrat soll bei Temperaturen unterhalb von 50°C erfolgen. Danach wird dem Dampfkreislauf der Kesselinhalt innerhalb von 60 Minuten auf eine Temperatur von 140°C gebracht und die Temperatur während 30 Minuten gehalten. Die Wasserstoffaufnahme beträgt in der Regel ca. 95 bis 100% der Theorie.

Anschliessend wird auf 60°C abgekühlt und nach geschlossenem Einlassventil der Wasserstoffdruck durch Entlasten ins Freie auf 1 bar reduziert. Das Ausspülen des Wasserstoffs im Kessel erfolgt durch 3maliges Aufpressen und Entlasten von jeweils 5 bar Stickstoff. Nun wird der Kesselinhalt über eine vorgewärmte Drucknutsche aus Hastelloy B® (Heisswasser-Heizung) in einen tarierten Transportbehälter abgepresst und mit Isopropanol nachgespült.

Das isopropanolische Filtrat enthält das Produkt. Dieses wird mittels Alkalilaugen auf einen pH-Wert von 9 bis 12 alkalisch gestellt. Die Hauptlösung sowie die Waschlösung werden vereinigt und einer Destillation unterworfen. Wenn das Lösungsmittel mehr oder weniger vollständig abdestilliert ist, kann das Reduktionsprodukt aus dem Rückstand isoliert werden. Es ist derart rein, dass es direkt ohne Isolierung weiterumgesetzt werden kann.

Der zuvor abfiltrierte Katalysator eignet sich gegebenenfalls nach Regenerierung für weitere Umsetzungen.

Die nach dem neuen Einstufenverfahren zugänglichen Produkte sind wertvolle Zwischenprodukte zur Herstellung von Farbstoffen und Farbpigmenten, z.B. durch Umsetzung mit Chloressigsäure zu den entsprechenden Thioglykolsäuren.

Folgende Beispiele veranschaulichen das neue Verfahren.

## Beispiel 1

In einem 2500 $cm^3$-Email-Autoklav — übrige Autoklavenausrüstung aus Hastelloy B® — werden 245,5 g (1 Mol) 2,5-Dichlorophenyl-1-sulfochlorid in 1100 ml Isopropanol mit 4 g Platin-Kohle-Katalysator (200 mg Pt) vorgelegt. Nach den Verdrängen der Luft durch 3maliges Aufpressen und Entlasten von jeweils 5 bar $N_2$ und dem Verdrängen des Stickstoffs auf analoge Weise mit Wasserstoff wird der Wasserstoffdruck über ein Druckreduzierventil auf 40 bar eingestellt. Nach der Dichtigkeitsprobe wird der Rührer mit 1200 $min^{-1}$ in Gang gesetzt. Die Reaktionstemperatur steigt innerhalb von 1 bis 2 Minuten von 20°C auf 50°C an und fällt innert weiteren 5 Minuten wieder auf 40°C ab. Die Wasserstoffaufnahme beträgt während dieser Zeit rund 1 Mol.

Anschliessend wird innert 50 Minuten annähernd linear auf 140°C aufgeheizt und die Temperatur während 30 Minuten bei 140°C gehalten. Nach dem Abkühlen wird der $H_2$-Druck ins Freie entlastet. Durch 3maliges Aufpressen und Entlasten von jeweils 5 bar $N_2$ wird der Wasserstoff aus dem Autoklaven gespült.

Vom Katalysator wird abfiltriert und das Filtrat mit 2,5 Mol 30%iger Natronlauge (250 Mol) versetzt.

Das erhaltene 2,5-Dichlorthiophenolat konnte nach dem Abdestillieren des Isopropanol-$H_2O$-Azeotrops ohne Isolierung praktisch quantitativ mit 1,05 Mol Chloressigsäure zur 2,5-Dichlorphenyl-1-thioglykolsäure umgesetzt werden.

Die Reinausbeute an 2,5-Dichlorthiophenol betrug während mehreren Versuchen zwischen 93 und 96% der Theorie.

## Beispiel 2

Durch Vorlegen von 122,8 g ($\frac{1}{2}$ Mol) 2,5-Dichlorphenyl-1-sulfochlorid in 600 ml Toluol mit 7 g feuchtem sulfoniertem Platin-Kohle-Katalysator (40 bis 50% Wassergehalt, 5% Pt i.Tr.) — Wasserstoffdruck 120 bis 155 bar, Aufheizen auf 150°C und Halten der Temperatur von 150°C während 6 Stunden — wurden ebenfalls Ausbeuten an 2,5-Dichlorthiophenol von 95% der Theorie erhalten.

Beispiele 3 bis 12

Verfährt man wie im Beispiel 1 beschrieben, jedoch unter Verwendung äquimolekularer Mengen der in Kolonne 2 der folgenden Tabelle aufgeführten Arylsulfochloride, so erhält man die entsprechenden in Kolonne 3 der Tabelle aufgeführten Arylthiole in der Regel in derselben Reinheit und in Ausbeuten von ebenfalls über 90% der Theorie.

TABELLE

| Bsp. Nr. | Arylsulfochloride | Arylthiole |
|---|---|---|
| 3 | 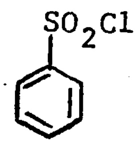 | Thiophenol |
| 4 | 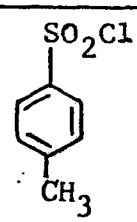 | 4-Methylthiophenol |
| 5 | 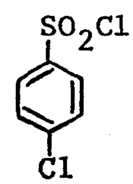 | 4-Chlorthiophenol |
| 6 | 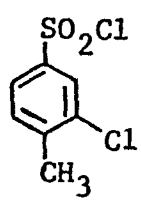 | 4-Methyl-3-chlorthiophenol |
| 7 | 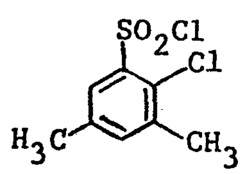 | 2-Chlor-3,5-dimethylthiophenol |
| 8 | 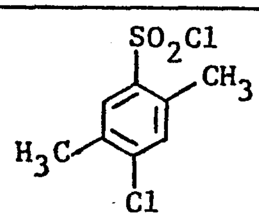 | 2,5-Dimethyl-4-chlorthiophenol |

| Bsp. Nr. | Arylsulfochloride | Arylthiole |
|---|---|---|
| 9 | 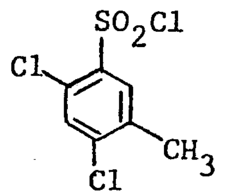 | 3-Methyl-4,6-dichlor-thiophenol |
| 10 | 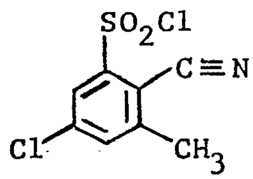 | 3-Chlor-5-methyl-6-cyanthiophenol |
| 11 | 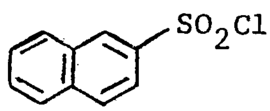 | 2-Thionaphthol |
| 12 | 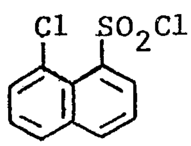 | 8-Chlor-1-thionaphthol |

**Patentansprüche**

1. Verfahren zur Herstellung von Arylthiolen durch katalytische Hydrierung von Arylsulfochloriden in einem protonischen oder aprotonischen, organischen Lösungsmittel bei erhöhtem Druck und unter erhöhten Temperaturen, dadurch gekennzeichnet, dass man in Gegenwart von Platin als Katalysator bei Temperaturen zwischen 100 und 180°C, mit Wasserstoff unter einem Druck von 2 bis 140 bar, hydriert.

2. Verfahren nach Anspruch 1, dadurch gekennzeichnet, dass man als Katalysator sulfidiertes Platin verwendet.

3. Verfahren nach Ansprüchen 1 und 2, dadurch gekennzeichnet, dass man die Hydrierung im Temperaturbereich zwischen 120 bis 180°C, insbesondere 140 bis 150°C, und einem Wasserstoffdruckbereich von 20 bis 120, insbesondere 30 bis 100 bar durchführt.

4. Verfahren nach Ansprüchen 1 bis 3, dadurch gekennzeichnet, dass man den Katalysator auf Trägern mit hoher spezifischer Oberfläche einsetzt.

5. Verfahren nach Anspruch 4, dadurch gekennzeichnet, dass man als Katalysatorträger Aktivkohle, Kieselgel, Aluminiumgel oder Magnesiagel verwendet.

6. Verfahren nach Ansprüchen 1 bis 5, dadurch gekennzeichnet, dass man als protonische Lösungsmittel die niederen aliphatischen Alkohole oder Dimethylformamid verwendet.

7. Verfahren nach Ansprüche 1 bis 6, dadurch gekennzeichnet, dass man als aprotonische Lösungsmittel Toluol, Xylole oder Chlorbenzole verwendet.

8. Verfahren nach Ansprüchen 1 bis 7, dadurch gekennzeichnet, dass man als Ausgangsprodukt Benzolsulfochlorid verwendet.

**Claims**

1. A process for the production of arylthiols by catalytic hydrogenation of arylsulfochlorides in a protic or aprotic organic solvent, characterised in that the hydrogenation is carried out at elevated pressure and temperature in the presence of platinum as catalyst, in the temperature range from 100°

to 180°C, with hydrogen, under a pressure of 2 to 140 bar.

2. A process according to claim 1, characterised in that sulfidised platinum is used as catalyst.

3. A process according to claims 1 and 2, characterised in that the hydrogenation is carried out in the temperature range from 120° to 180°C, in particular from 140° to 150°C, and in a hydrogen pressure range from 20 to 120 bar, in particular from 30 to 100 bar.

4. A process according to claims 1 to 3, characterised in that the catalyst is used on a carrier having a high specific surface area.

5. A process according to claim 4, wherein the carrier is activated charcoal, silica gel, aluminium gel, or magnesia gel.

6. A process according to claims 1 to 5, characterised in that a lower aliphatic alcohol or dimethyl formamide is used as protic solvent.

7. A process according to claims 1 to 6, characterised in that toluene, a zylene or a chlorobenzene is used as aprotic solvent.

8. A process according to claims 1 to 7, characterised in that benzenesulfochloride is used as starting material.

**Revendications**

1. Procédé pour la préparation de thiophénols par hydrogénation catalytique des arylsulfochlorures dans un solvant organique protonique ou aprotique, caractérisé par le fait qu'on procède à l'hydrogénation sous pression élevée et à température élevée, en présence de platine comme catalyseur, les températures étant comprises entre 100° et 180°C, avec de l'hydrogène sous une pression de 2 à 140 bars.

2. Procédé selon la revendication 1, caractérisé par le fait qu'on utilise comme catalyseur de platine sulfuré.

3. Procédé selon les revendications 1 et 2, caractérisé par le fait qu'on effectue l'hydrogénation dans une zone de température comprise entre 120° et 180°C, en particulier entre 140° et 150°C et dans une zone de pression d'hydrogène allant de 20 à 120 bars, en particulier de 30 à 100 bars.

4. Procédé selon l'une quelconque des revendications 1 à 3, caractérisé par le fait qu'on utilise le catalyseur sur des supports ayant une surface spécifique élevée.

5. Procédé selon la revendication 4, caractérisé par le fait qu'on utilise comme support de catalyseur le charbon actif, le gel de silice, le gel d'aluminium ou le gel de magnésie.

6. Procédé selon l'une quelconque des revendications 1 à 5, caractérisé par le fait qu'on utilise comme solvant protonique les alcools aliphatiques inférieurs ou le diméthylformamide.

7. Procédé selon l'une quelconque des revendications 1 à 6, caractérisé par le fait qu'on utilise comme solvant aprotique le toluène, les xylènes ou les chlorobenzénes.

8. Procédé selon l'une quelconque des revendications 1 à 7, caractérisé par le fait qu'on utilise comme produit de départ le benzènesulfochlorure.